(19)

Europäisches
Patentamt
European
Patent Office
Office européen
des brevets

(11) **EP 2 965 762 A1**

(12) **EUROPEAN PATENT APPLICATION**

(43) Date of publication:
**13.01.2016 Bulletin 2016/02**

(21) Application number: **14197294.3**

(22) Date of filing: **15.09.2011**

(51) Int Cl.:
*A61K 31/565* (2006.01)     *A61K 31/585* (2006.01)
*A61K 31/57* (2006.01)     *A61P 15/00* (2006.01)
*A61K 31/05* (2006.01)     *A61K 31/566* (2006.01)
*A61K 31/567* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.09.2010 BR PI1003661**

(62) Document number(s) of the earlier application(s) in
accordance with Art. 76 EPC:
**11824381.5 / 2 617 425**

(71) Applicant: **Libbs Farmacêutica Ltda.
01140-050 - São Paulo (BR)**

(72) Inventor: **Da Silva Maia Filho, Hugo
40210-340 São Paulo (BR)**

(74) Representative: **KATZAROV S.A.
European Patent Attorneys
19, rue des Epinettes
1227 Genève (CH)**

Remarks:
•Claims 16-23 are deemed to be abandoned due to
non-payment of the claims fees (Rule 45(3) EPC).
•This application was filed on 11-12-2014 as a
divisional application to the application mentioned
under INID code 62.

(54) **Pharmaceutical combination of resveratrol and progestin to treat and/or prevent myoma
and/or endometriosis**

(57)    The present invention relates to a combination of resveratrol with progestogen, and medicaments and pharmaceutical compositions containing the same, which are useful in the treatment and/or prevention of myoma and/or endometriosis, providing their size reduction and the control of related symptoms. Said association may additionally comprise an estrogen component. A treatment method and a kit are also objects of this invention.

FIGURE 1

**Description**

**Field of the Invention**

The present invention relates to a combination of resveratrol with progestogens, to medicaments and pharmaceutical compositions containing the same and their use in the treatment or prevention of myoma and/or endometriosis and in the control of related symptoms. A treatment method and kit are also objects of this invention.

**Background of the Invention**

The endometrium is the tissue layer comprising glands and stroma covering the uterus. Scaling of that tissue is responsible for menstruation at the end of the menstrual cycle.

Endometriosis is a chronic disease characterized by the presence of endometrial tissue in other parts of the body other than the uterus. There are many theories about the causes of this disease. The main one states that endometrial cells are transported to other parts of the body during menstruation through the Fallopian tubes. It is also believed that it has genetic causes.

Endometriosis can occur in various forms, classified according to size and location; for example, it can be peritoneal or extraperitoneal, deep, of pelvic adherence, adenomyosis and endometrial cysts in the ovary.

The endometrial implant responds as the normal endometrial tissue to variations of progesterone and estrogen concentrations, and it grows like this tissue during the menstrual cycle.

The presence of these implants causes many symptoms, including chronic pelvic pain and dysmenorrhea, and it is often associated with infertility, although this association is not completely elucidated.

In order to treat this condition, surgeries for removing implants and the administration of several compounds are made, separately or together, but most part of these methods is not curative.

The drug treatments available are limited to the growth inhibition of endometrial implant, particularly by inhibiting estrogen production.

Although the drug treatments halt the implant growth, they do not allow its reduction, leaving the surgical removal as the only viable solution, i.e., the use of undesirable invasive methods.

Another common disease of the female genital tract is the presence of myomas. Many patients with endometriosis also have myomas. These are benign tumors that occur in smooth muscles, which may be present in several human organs, the most common being uterine myoma.

The cause of myoma development (as well as of endometriosis) has not been completely elucidated. The state of the art commonly proposes that it is originated from a single cell, which growths disorderly, thus forming the myoma. It is also mentioned that the presence of these tumors is correlated with the exposure to high levels of estrogen.

The myomas are also classified according to their location, and they can be subserosal, intramural or submucosal. Depending on the site, size and number, the symptoms may include abdominal discomfort, dysmenorrhea, high urinary frequency or retention and, in some cases, infertility.

Myoma, in most cases, is treated only when symptoms appear, since this is a benign tumor and it is commonly asymptomatic.

When treatment is needed, it is conducted through medication, by administering anti-inflammatory drugs and gonadotropin-releasing hormone (GnRH) analogues, which reduce estrogen release by the ovaries, reducing their growth. On the other hand, surgical method, including myomectomy and hysterectomy, can be definitive.

Although the surgical methods of diagnosis and treatment are more effective, they involve risks inherent to any invasive procedure, which lead to poor compliance, and recurrences are common in less than five years.

On the other hand, although the symptomatic clinical treatment for endometriosis and myoma reduce pain and immediate discomfort, they are limited by about six months due to potential negative effects on bone density.

As an additional characteristic, the drug treatment typically results only in the stagnation of structures growth, neither allowing the regression and potential elimination of cysts nor efficiently reducing all symptoms of discomfort, particularly related to pain.

The pelvis, which comprises the uterus, uterine tubes, ovaries, vagina, bladder and rectum, is the lower part of the trunk, located below the abdomen and between the hip bones. Pain in the pelvic region or pelvic pain is common in women, but it is not always caused by problems related to the reproductive system. Other causes of pelvic pain are related to the intestines, urinary tract or even psychological factors. The type and intensity of pain vary and its cause can be difficult to identify.

Another type of pain that often affects women is dysmenorrhea, an abdominal pain caused by uterine contractions that occur during menstruation. This disorder is named primary dysmenorrhea when no cause is verified and secondary dysmenorrhea when the cause is a gynecological disorder. Primary dysmenorrhea is common, possibly affecting more than 50% of women. It is severe in about 5 to 15%.

Headache, nausea, constipation or diarrhea, vomiting and urgency to urinate frequently are associated with hormonal phenomena in women.

Generally, pain can be relieved with the administration of non-steroidal anti-inflammatory drugs (for example, ibuprofen, naproxen and mefenamic acid). These medicaments are effective when they start to be taken 2 days before menstruation and are administered 1 or 2 days during menstruation. Nausea and vomiting may be relieved with the administration of an antiemetic medicament (against vomiting), but these symptoms usually disappear without treatment when colic stops.

When pain persists and interferes with the normal activity of women, ovulation can be suppressed with low doses of oral contraceptives that contain estrogen and progestin or with medroxyprogesterone (long-action). When these treatments fail, additional tests may be needed, such as laparoscopy.

The treatment of secondary dysmenorrhea depends on its cause. A narrow cervical canal can be surgically enlarged, commonly providing three to six months of relief. When treatment is not successful and pain is intense, the nerves section that innervates the uterus is occasionally useful. Complications include injury of other pelvic organs (e.g., ureters).

Therefore, the clinical diagnosis from symptoms is important and, in many cases, the control of symptoms is enough.

Resveratrol is a known polyphenol and, according to literature, it is used as a nutrition component, since its main common source is grape. This compound can be obtained from natural or synthetic sources, and it is described in the state of the art as an estrogen antagonist used in the treatment of cardiovascular diseases, since it has the ability to decrease levels of low-density lipoproteins (LDL), decreasing their production in the liver and preventing its oxidation and it increases the levels of high-density lipoproteins (HDL), favoring its production in the liver (Harikumar et al: Resveratrol - A multitargeted agent for age-associated chronic diseases, Cell Cycle 7:8, 1020-1035 April 15th, 2008).

Moreover, many recent studies are investigating whether resveratrol has antiproliferative activity on some neoplasms, such as those occurring in breast, ovary, colon and prostate. This activity would occur since neoplasms express great amounts of estrogen receptor on their surfaces. Nevertheless, more recent studies have shown that resveratrol has low activity on estrogen receptor when compared to other phytoestrogens and has no effect on cell proliferation in a model system used in the research of synthesis of steroid hormones (Chen et. Al.: Effects of genistein, resveratrol, and quercetin on steroidogenesis, Journal of Endocrinology (2007) 192, 527-537). As other medicaments available, even having antiproliferative action, the state of the art does not teach that resveratrol is able to cause regression of these neoplasms.

Progestogens, also known as progestagens or gestagens, are a group of hormones including progesterone. Typically, they are used alone or in combination with estrogen in oral contraceptives.

Among the oral contraceptives that use a combination of progestogen and estrogen, there are the monophasic of first, second and third generation, in which the administered doses are always the same, and the biphasic and triphasic, in which the doses administered change to mimic the physiological release of these hormones.

There are many adverse effects related to the use of progestagens, such as increased appetite and slow weight gain, depression, fatigue, tiredness, acne and diabetogenic effect, among others. The association between progestogens and estrogens also cause side effects, such as breast tenderness, headache and arterial hypertension. The continuous use causes pain and blood escape. These effects can vary, depending on the dose of estrogen, the type and dose of progestagen or administration route.

The oral contraceptives of extended regime are used to treat hyperproliferative uterine disorders, which are effective for treating pain and bleeding caused by endometriosis and myomas. However, this treatment is currently not effective for decreasing the size of these abnormalities and its continuous use causes many unpleasant symptoms, such as blood escape.

Thus, there remains the need for new treatments, which not only prevent the growth of abnormalities (endometriosis and myoma), but are also effective at regression of their size and in the control of associated symptoms, eliminating the use of complimentary methods, such as invasive methods.

## Brief Description of the Figures

The present invention is illustrated by the following figures:

Figure 1 shows a graph of effects on the uterine volume in a patient who has received, for 2 to 3 months, the combination according to the present invention, in one of its embodiments, containing 3 mg drospirenone, 0.03 mg of ethinyl estradiol and 50 mg of resveratrol.

Figures 2A and 2B are ultrasounds, respectively before and after, showing reduction of submucosal myoma in a patient who received, for 2 to 3 months, the combination according to the present invention in one of its embodiments, containing 3 mg of drospirenone, 0.03 mg of ethinyl estradiol and 50 mg of resveratrol.

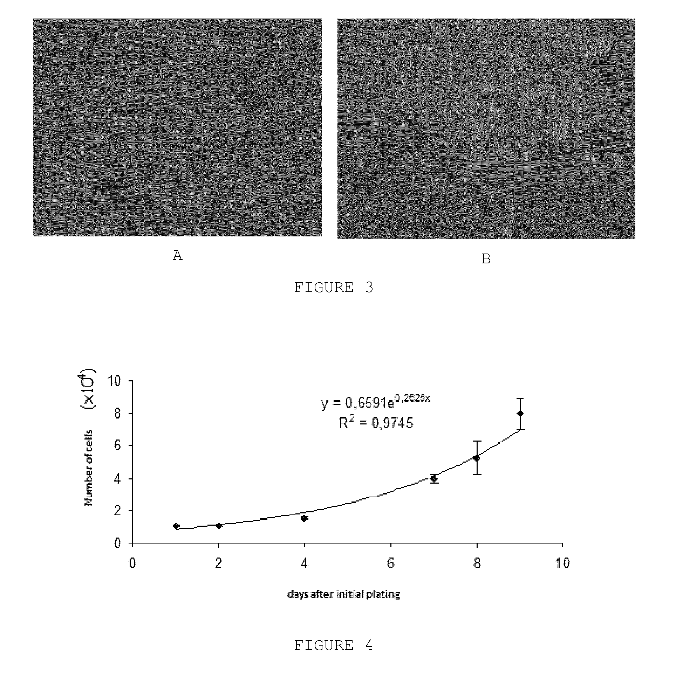

Figure 3 shows the cells obtained 24 h after isolation. The primary culture obtained from endometrial human tissue after 24 h of isolation: A - cells of the tissue sample 1. 100x. B - cells of the tissue sample 2. 100x.

Figure 4 shows the growth curve obtained from tissue sample 1 during 9 days of plating.

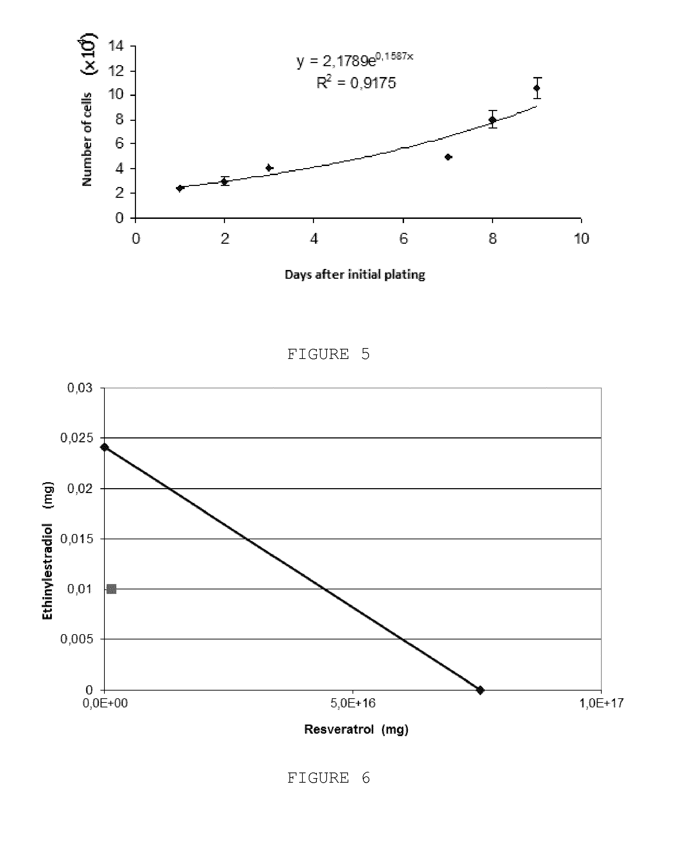

Figure 5 shows the growth curve obtained from tissue sample 2 during 9 days of plating.

Figure 6 presents an isobologram delineated from the obtained results.

## Description of the invention

In an attempt to find an effective alternative for the treatment and/or prevention of these abnormalities that affect a large number of women, the Applicant has developed a specific combination of compounds that synergistically provides simultaneous effects, particularly myoma size reduction and/or endometrial implant (endometriosis).

According to the present invention, "combination" means that the active principles are available in a single dosage unit or in separate units for combined or sequential administration.

"Treatment efficacy" means growth stopping, myoma and/or endometrial implant (endometriosis) reduction and control of associated symptoms.

The present invention encompasses "reduce" in any of its aspects, i.e., any noticeable decrease in terms of size, volume, area, etc.

"Effectiveness in prevention" means the prevention of the onset or recurrent onset of myomas and/or endometriosis and/or the control of symptoms commonly associated with them.

"Control of symptoms" means the improvement in the general symptomatological scenario according to the patient's perception.

According to the present invention, "endometriosis" comprises one or more of all the different manifestations, for example, peritoneal, extraperitoneal, deep, pelvic of adherence, adenomyosis and endometrioid cysts in the ovary.

"Synergic" means that the effects are greater than the simple sum of the individual effects.

"Simultaneous" means that one or more manifestations or related symptoms are treated at the same time, without the use of additional drug treatments.

The symptoms according to the present invention may include one or more of any physical manifestations that impair the patient's quality of life, such as pelvic pain, pain during intercourse or during the menstrual cycle (dysmenorrhea), blood escape and premenstrual syndrome.

Thus, the present invention relates to a combination of resveratrol with progestagens, particularly gestodene, desogestrel, levonogestrel, drospirenone and dienogest, optionally in the presence of an estrogen, particularly ethinyl estradiol, estradiol, estradiol valerate or mixtures thereof.

The present invention includes the compound resveratrol in any of its forms, such as isomers, salts, hydrates, solvates, derivatives, etc.

The isomers can be *cis* or *trans,* particularly *trans.*

The amount of reverastrol per dosage unit can vary between 15 mg and 10,000, particularly from 15 to 100 mg. This amount can vary according to the obtainment source and the physical-chemical properties of the compound obtained, for example, purity, solubility, etc. More particularly, an amount of about 30 mg per dosage unit is used.

The quantities per dosage unit of gestodene range from 35 mcg to 115 mcg, of drospirenone range from 1.5 mg to 4.5 mg, of dienogest range from 1 mg to 4 mg, of levonogestrel range from 40 to 70 mg and of desogestrel range from 75 mcg to 225 mcg. Other progestogens may be used in therapeutically equivalent amounts.

More particularly, the combination additionally comprises from 0.001 to 40 mcg of ethinyl estradiol, particularly from 0.1 to 40 mcg. Other estrogens or estrogen mixtures may be used in therapeutically equivalent amounts.

When progestogens are used in low doses, in a condition that they are not effective for contraception, an effective combination is advantageously obtained in the treatments described herein, without preventing pregnancy.

The estrogen component significantly contributes to improve the bleeding pattern of patients and to the symptoms control.

Another object of the present invention consists of a pharmaceutical composition containing the combination described herein and pharmaceutically acceptable excipients. The appropriate excipients can be selected among those known in the art, particularly for the preparation of tablets. A reference work for the formulation of such pharmaceutical forms is Remington's Pharmaceutical Sciences, of Mack Publishing American publisher.

Without causing any limitation, the following are examples of pharmaceutically acceptable excipients: stearic acid, ethyl alcohol, starch, sodium croscarmellose, silicon dioxide, dl-$\alpha$-tocopherol, sodium edetate, magnesium stearate, lactose and povidone. The compositions can also include coating forming excipients, colorants, etc.

A third object according to the present invention consists of a kit for delivering the combination to patients. Said kit comprises 20 to 30 dosage forms, particularly 21, 24 or 28 in a package, along with use instructions. The package may further contain additional tablets comprising resveratrol or placebo, for example, 1 to 10, particularly 7 or 4 tablets.

The combination according to the present invention is effective in the treatment or prevention of endometriosis and/or myoma not only by preventing the growth of such abnormalities, but also by providing a statistically significant reduction of the same.

Said combination also provides control of the symptoms associated with these diseases and it further provides a contraceptive effect in women of childbearing age or a hormonal replacement for women that need it, for example, in perimenopause or menopause stage. In this sense, the dosages of progestogens used in the combination according to the present invention are those that provide contraceptive effect.

Surprisingly, the combination was also simultaneously effective in the treatment of these manifestations, which may prevent the use of additional medicaments.

Hence, another object of the present invention relates to the use of resveratrol and progestogen in the preparation of a medicament useful in the treatment (reduction) and/or prevention of myoma and/or endometriosis and/or control of associated symptoms.

The present invention further relates to a method to treat (reduce) and/or prevent myoma and/or endometriosis, which consists in providing a combination during 20 to 30 days, particularly 21 or 24 days, or continuously, to a patient in need of such combination according to the present invention. The methods can be followed by 1 to 10 days, particularly 7 or 4 days of interruption or administration of resveratrol alone. The delivery can be simultaneous, into a single administration unit or in different units, or sequential, in separate administration units.

## EXAMPLES

The following presented examples are merely illustrative of the realization of the present invention. These examples do not represent any limitation to the scope of this invention.

## EXAMPLE 1

## FORMULATIONS

There were prepared control formulations and formulations according to the present invention, in an amount sufficient to test the effectiveness.

Formulations containing only progestogen or progestogen and estrogen, used as control or in the combination in accordance with the present invention, are in accordance with those available on the market.

## EXAMPLE 2

## EFFICACY TESTS

To illustrate the combination effectiveness in accordance with the present invention, resveratrol combinations were prepared in amounts of 30 mg and 50 mg, with:

    A - 75 mcg of gestodene and 30 mcg of ethinyl estradiol;
    B - 3 mg of drospirenone and 30 mcg of ethinyl estradiol, and
    C - 52 mg of levonogestrel (intrauterine system).

In the embodiments A and B above, oral administration forms (tablets) were used, and in embodiment C, progestogen was used as an intrauterine system along with resveratrol in an oral administration form (tablets).

In order to verify the simultaneous effects provided by the combination in accordance with the present invention, a pilot test was conducted with 18 female patients, aged between 27 and 43 years, of which: 3 patients had adenomyosis and myoma; 2 patients had adenomyosis; 8 patients had myoma; 1 patient had endometrial cyst; 2 patients had endometriosis; and 2 patients had myoma and endometriosis.

Each patient received one of the combination types described above (A to C) daily.

The uterine volume was measured at different times for each patient (between 2 and 9 months). It was observed that 17 patients had a significant percentage decrease in uterine volume.

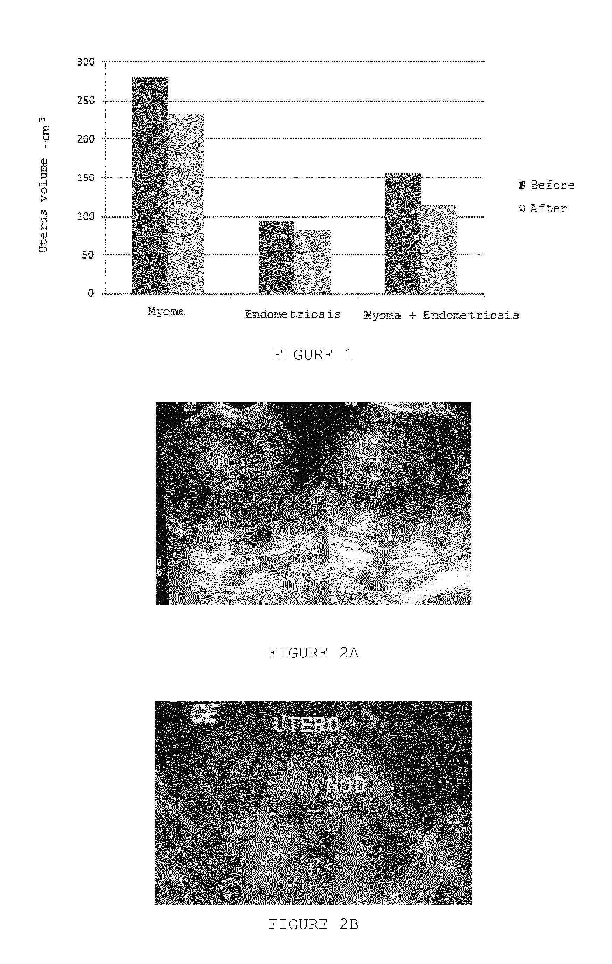

Figure 1 shows the effects on the uterine volume in one of the patients who received 3 mg drospirenone, 0.03 mg of ethinyl estradiol and 50 mg of resveratrol, during almost 3 months.

The myoma volume was evaluated by vaginal ultrasonography, and the intensity of symptoms, such as bleeding and pain, was assessed by a questionnaire answered before and after the treatment.

Figures 2A and 2B are ultrasounds showing the submucosal myoma reduction in one of the patients who received 3 mg drospirenone, 0.03 mg of ethinyl estradiol and 50 mg of resveratrol, during almost 3 months.

There was no appearance of new abnormalities in all patients, and the combination in accordance with the present invention proved to be effective to reduce pelvic pain and cause amenorrhea. It was observed, in patients with myoma, a decrease of approximately 30% by volume between the second and the fourth month of use.

The normalization of bleeding standard ,i.e. no irregular uterine bleeding, was also observed.

## EXAMPLE 3

## UTERUS VOLUME

## INTRAMURAL MYOMA, ADENOMYOSIS AND ENDOMETRIOSIS

An experiment was carried out with 30 patients treated with combinations of drospirenone, ethinyl estradiol and resveratrol, as well as with combinations of gestinol and resveratrol, evaluating the volume of the uterus before and after the treatment.

**Table 1**

| Measurements of the uterus volume Patients with intramural myoma, adenomyosis and endometriosis Evaluation before and after the treatment using combinations of drospirenone, ethinyl estradiol and resveratrol and combinations of gestinol and resveratrol | | | | | |
|---|---|---|---|---|---|
| Pre-volume | Post-volume | Treatment duration | Pathology | Compounds | % of reduction |
| 621 | 583 | 3 months | Myoma | Drospirenone Ethinyl estradiol Resveratrol | 6.11916 |
| 239 | 196 | 1 month | Myoma | Drospirenone Ethinyl Estradiol Resveratrol | 17.9916 |
| 143 | 134 | 2 months | Endometriosis | Drospirenone Ethinyl Estradiol Resveratrol | 6.29371 |
| 157 | 121 | 3 months | Adenomyosis | Drospirenone Ethinyl Estradiol Resveratrol | 22.9299 |
| 87 | 65 | 6 months | Endometriosis | Drospirenone Ethinyl Estradiol Resveratrol | 25.2874 |
| 98 | 85 | 1 month | Endometriosis | Drospirenone Ethinyl Estradiol Resveratrol | 13.2653 |
| 172 | 160 | 1 month | Myoma | Drospirenone Ethinyl Estradiol Resveratrol | 6.97674 |
| 243 | 218 | 9 months | Myoma | Drospirenone Ethinyl Estradiol Resveratrol | 10.2881 |
| 100 | 80 | 1 month | Endometriosis | Drospirenone Ethinyl Estradiol Resveratrol | 20 |
| 609 | 232 | 3 months | Myoma | Drospirenone Ethinyl Estradiol Resveratrol | 61.9048 |
| 39 | 28 | 2 months | Endometriosis | Drospirenone Ethinyl Estradiol Resveratrol | 28.2051 |
| 244 | 188 | 1 month | Myoma | Drospirenone Ethinyl Estradiol Resveratrol | 22.9508 |
| 134 | 60 | 12 months | Endometriosis | Drospirenone Ethinyl Estradiol Resveratrol | 55.2239 |
| 82 | 73 | 3 months | Endometriosis | Drospirenone Ethinyl Estradiol Resveratrol | 10.9756 |
| 213 | 130 | 2 months | Myoma | Drospirenone Ethinyl Estradiol Resveratrol | 38.9671 |

(continued)

| Measurements of the uterus volume Patients with intramural myoma, adenomyosis and endometriosis Evaluation before and after the treatment using combinations of drospirenone, ethinyl estradiol and resveratrol and combinations of gestinol and resveratrol | | | | | |
|---|---|---|---|---|---|
| Pre-volume | Post-volume | Treatment duration | Pathology | Compounds | % of reduction |
| 150 | 110 | 6 months | Adenomyosis | Drospirenone Ethinyl Estradiol Resveratrol | 26.6667 |
| 652 | 414 | 2 months | Myoma | Drospirenone Ethinyl Estradiol Resveratrol | 36.5031 |
| 112 | 81 | 2 months | Adenomyosis | Drospirenone Ethinyl Estradiol Resveratrol | 27.6786 |
| 217 | 159 | 3 months | Myoma | Gestinol Resveratrol | 26.7281 |
| 305 | 235 | 5 months | Myoma | Gestinol Resveratrol | 22.9508 |
| 62 | 58 | 2 months | Endometriosis | Gestinol Resveratrol | 6.45161 |
| 141 | 121 | 3 months | Myoma | Gestinol Resveratrol | 14.1844 |
| 133 | 126 | 6 months | Endometriosis | Gestinol Resveratrol | 5.26316 |
| 71 | 37 | 3 months | Endometriosis | Gestinol Resveratrol | 47.8873 |
| 141 | 93 | 3 months | Endometriosis | Gestinol Resveratrol | 34.0426 |
| 105 | 86 | 3 months | Endometriosis | Gestinol Resveratrol | 18.0952 |
| 125 | 85 | 3 months | Endometriosis | Gestinol Resveratrol | 32 |
| 70 | 58 | 3 months | Myoma | Gestinol Resveratrol | 17.1429 |
| 245 | 137 | 3 months | Adenomyosis | Gestinol Resveratrol | 44.0816 |
| 70 | 58 | 3 months | Myoma | Gestinol Resveratrol | 17.1429 |

As can be observed, the combination of drospirenone, ethinyl estradiol and resveratrol led to a reduction of 61% of the myoma and up to 55% of endometriosis and the combination of gestinol and resveratrol resulted in a decrease of up to 26% of myomas and 47% of endometrial implants.

In the comparative studies with three patients A, B and C, showing intramural myoma treated only with resveratrol, there was observed a 32% increase for patient A, treated during 12 months; 27% increase for patient B, treated during 3 months; and 22% increase for patient C, treated during 1 month.

## EXAMPLE 4

## ASSESSMENT OF SYMPTOMS OF DYSMENORRHOEA AND BLEEDING

In an experiment aimed at assessing the symptoms of dysmenorrhea and bleeding, the 83 patients with endometriosis, myoma or adenomyosis were asked to evaluate said symptoms in scales of 0 to 3, before and after the treatment with combinations of drospirenone, ethinyl estradiol and resveratrol, and with combinations of gestinol and resveratrol.

For dysmenorrhea, the following scale was used: 0 - no pain; 1 - low pain; 2 - moderate pain; and 3 - strong pain. For bleeding, the following scale was used: 0 - no bleeding; 1 - low bleeding; 2 - moderate bleeding; and 3 - strong bleeding.

Table 2

| Assessment of dysmenorrhea and bleeding symptoms in 83 patients in 0-3 scale. | | | | |
|---|---|---|---|---|
| Evaluated symptom | Before treatment | After treatment | Compounds | Pathology |
| Dysmenorrhea | 3 | 0 | Drospirenone Ethinyl Estradiol Resveratrol | Myoma |
| Dysmenorrhea | 3 | 0 | Drospirenone Ethinyl Estradiol Resveratrol | Endometriosis |
| Dysmenorrhea | 3 | 1 | Drospirenone Ethinyl Estradiol Resveratrol | Endometriosis |
| Dysmenorrhea | 3 | 0 | Drospirenone Ethinyl Estradiol Resveratrol | Endometriosis |
| Dysmenorrhea | 3 | 1 | Drospirenone Ethinyl Estradiol Resveratrol | Myoma |
| Dysmenorrhea | 3 | 0 | Drospirenone Ethinyl Estradiol Resveratrol | Endometriosis |
| Dysmenorrhea | 3 | 0 | Drospirenone Ethinyl Estradiol Resveratrol | Myoma |
| Dysmenorrhea | 3 | 1 | Drospirenone Ethinyl Estradiol Resveratrol | Myoma |
| Dysmenorrhea | 3 | 0 | Drospirenone Ethinyl Estradiol Resveratrol | Endometriosis |
| Dysmenorrhea | 3 | 0 | Drospirenone Ethinyl Estradiol Resveratrol | Myoma |
| Dysmenorrhea | 3 | 0 | Drospirenone Ethinyl Estradiol Resveratrol | Adenomyosis |
| Dysmenorrhea | 3 | 0 | Drospirenone Ethinyl Estradiol Resveratrol | Adenomyosis |
| Dysmenorrhea | 3 | 1 | Drospirenone Ethinyl Estradiol Resveratrol | Myoma |
| Dysmenorrhea | 3 | 1 | Drospirenone Ethinyl Estradiol Resveratrol | Myoma |
| Dysmenorrhea | 3 | 0 | Drospirenone Ethinyl Estradiol Resveratrol | Myoma |
| Dysmenorrhea | 2 | 0 | Drospirenone Ethinyl Estradiol Resveratrol | Myoma |
| Dysmenorrhea | 2 | 0 | Drospirenone Ethinyl Estradiol Resveratrol | Myoma |
| Dysmenorrhea | 3 | 0 | Drospirenone Ethinyl Estradiol Resveratrol | Adenomyosis |
| Dysmenorrhea | 3 | 0 | Gestinol Resveratrol | myoma |
| Dysmenorrhea | 3 | 0 | Gestinol Resveratrol | Myoma |
| Dysmenorrhea | 3 | 0 | Gestinol Resveratrol | Endometriosis |
| Dysmenorrhea | 3 | 2 | Gestinol Resveratrol | Myoma |
| Dysmenorrhea | 3 | 0 | Gestinol Resveratrol | Myoma |
| Dysmenorrhea | 3 | 1 | Gestinol Resveratrol | Endometriosis |

(continued)

| Assessment of dysmenorrhea and bleeding symptoms in 83 patients in 0-3 scale. | | | | |
|---|---|---|---|---|
| Evaluated symptom | Before treatment | After treatment | Compounds | Pathology |
| Dysmenorrhea | 3 | 0 | Gestinol Resveratrol | Endometriosis |
| Dysmenorrhea | 3 | 0 | Gestinol Resveratrol | Endometriosis |
| Dysmenorrhea | 3 | 1 | Gestinol Resveratrol | Endometriosis |
| Dysmenorrhea | 3 | 1 | Gestinol Resveratrol | Endometriosis |
| Dysmenorrhea | 3 | 1 | Gestinol Resveratrol | Endometriosis |
| Dysmenorrhea | 3 | 1 | Gestinol Resveratrol | Endometriosis |
| Dysmenorrhea | 3 | 0 | Gestinol Resveratrol | Endometriosis |
| Dysmenorrhea | 3 | 0 | Gestinol Resveratrol | Endometriosis |
| Dysmenorrhea | 3 | 0 | Gestinol Resveratrol | Myoma |
| Dysmenorrhea | 3 | 0 | Gestinol Resveratrol | Myoma |
| Dysmenorrhea | 2 | 0 | Gestinol Resveratrol | Myoma |
| Dysmenorrhea | 3 | 1 | Gestinol Resveratrol | Endometriosis |
| Dysmenorrhea | 2 | 0 | Gestinol Resveratrol | Myoma |
| Dysmenorrhea | 3 | 1 | Gestinol Resveratrol | Adenomyosis |
| Dysmenorrhea | 3 | 1 | Gestinol Resveratrol | Adenomyosis |
| Dysmenorrhea | 3 | 0 | Gestinol Resveratrol | Endometriosis |
| Bleeding | 3 | 0 | Drospirenone Ethinyl Estradiol Resveratrol | Myoma |
| Bleeding | 2 | 0 | Drospirenone Ethinyl Estradiol Resveratrol | Endometriosis |
| Bleeding | 3 | 0 | Drospirenone Ethinyl Estradiol Resveratrol | Endometriosis |
| Bleeding | 3 | 0 | Drospirenone Ethinyl Estradiol Resveratrol | Endometriosis |
| Bleeding | 3 | 0 | Drospirenone Ethinyl Estradiol Resveratrol | Myoma |
| Bleeding | 3 | 0 | Drospirenone Ethinyl Estradiol Resveratrol | Endometriosis |
| Bleeding | 3 | 0 | Drospirenone Ethinyl Estradiol Resveratrol | Myoma |
| Bleeding | 2 | 1 | Drospirenone Ethinyl Estradiol Resveratrol | Myoma |
| Bleeding | 3 | 0 | Drospirenone Ethinyl Estradiol Resveratrol | Endometriosis |
| Bleeding | 2 | 0 | Drospirenone Ethinyl Estradiol Resveratrol | Endometriosis |
| Bleeding | 3 | 0 | Drospirenone Ethinyl Estradiol Resveratrol | Adenomyosis |
| Bleeding | 3 | 0 | Drospirenone Ethinyl Estradiol Resveratrol | Adenomyosis |

(continued)

| Assessment of dysmenorrhea and bleeding symptoms in 83 patients in 0-3 scale. | | | | |
|---|---|---|---|---|
| Evaluated symptom | Before treatment | After treatment | Compounds | Pathology |
| Bleeding | 3 | 0 | Drospirenone Ethinyl Estradiol Resveratrol | Myoma |
| Bleeding | 2 | 1 | Drospirenone Ethinyl Estradiol Resveratrol | Myoma |
| Bleeding | 2 | 0 | Drospirenone Ethinyl Estradiol Resveratrol | Endometriosis |
| Bleeding | 2 | 0 | Drospirenone Ethinyl Estradiol Resveratrol | Myoma |
| Bleeding | 3 | 1 | Drospirenone Ethinyl Estradiol Resveratrol | Myoma |
| Bleeding | 3 | 0 | Drospirenone Ethinyl Estradiol Resveratrol | Adenomyosis |
| Bleeding | 2 | 0 | Gestinol Resveratrol | Myoma |
| Bleeding | 3 | 0 | Gestinol Resveratrol | Myoma |
| Bleeding | 3 | 0 | Gestinol Resveratrol | Adenomyosis |
| Bleeding | 3 | 0 | Gestinol Resveratrol | Endometriosis |
| Bleeding | 1 | 0 | Gestinol Resveratrol | Myoma |
| Bleeding | 3 | 0 | Gestinol Resveratrol | Endometriosis |
| Bleeding | 3 | 2 | Gestinol Resveratrol | Myoma |
| Bleeding | 3 | 0 | Gestinol Resveratrol | Myoma |
| Bleeding | 3 | 0 | Gestinol Resveratrol | Myoma |
| Bleeding | 3 | 0 | Gestinol Resveratrol | Endometriosis |
| Bleeding | 3 | 0 | Gestinol Resveratrol | Endometriosis |
| Bleeding | 3 | 0 | Gestinol Resveratrol | Endometriosis |
| Bleeding | 3 | 1 | Gestinol Resveratrol | Adenomyosis |
| Bleeding | 3 | 1 | Gestinol Resveratrol | Myoma |
| Bleeding | 2 | 0 | Gestinol Resveratrol | Endometriosis |
| Bleeding | 3 | 1 | Gestinol Resveratrol | Adenomyosis |
| Bleeding | 3 | 0 | Gestinol Resveratrol | Endometriosis |
| Bleeding | 3 | 0 | Gestinol Resveratrol | Endometriosis |
| Bleeding | 3 | 0 | Gestinol Resveratrol | Endometriosis |
| Bleeding | 3 | 1 | Gestinol Resveratrol | Myoma |
| Bleeding | 3 | 0 | Gestinol Resveratrol | Endometriosis |
| Bleeding | 3 | 0 | Gestinol Resveratrol | Myoma |
| Bleeding | 3 | 0 | Gestinol Resveratrol | Myoma |
| Bleeding | 2 | 0 | Gestinol Resveratrol | Myoma |
| Bleeding | 3 | 0 | Gestinol Resveratrol | Endometriosis |

It was observed that the combination according to the present invention significantly reduces the symptoms evaluated.

## EXAMPLE 5

## EVALUATION OF THE ACTION OF ISOLATED AND CONJUGATED COMPOUNDS

### Objectives

Establish the primary culture of stromal cells extracted from human endometrium tissue and assess the synergism of estrogen/progestogen with phytosterol (trans-3,5,4"-trihidroxystilbene).

### Material and Methods

### Substance-test:

187A: Dienogest - synthetic progesterone used in birth control pills and studied in treatments of endometriosis (fixed dose of 2 mg) - conjugated with ethinyl estradiol - synthetic estrogen used in contraceptive pills (doses ranging from 0 and 40 $\mu$g).
187B: Resveratrol - phytoalexin produced by various plants, such as grape, peanut and blackberry, with antioxidant, anticoagulant, anti-inflammatory and antiproliferative activities.
187C: Drug combination - Dienogest (2 mg) + Ethinyl estradiol + Resveratrol
187D: Combination of dienogest (2 mg) + Resveratrol.
187E: Dienogest alone.
187F: Ethinyl estradiol alone.

### Reference substance:

Positive control (Dan), Danazol (Ladogal ®) commercially available:

Negative control: stromal cells of endometrioma incubated with 200 $\mu$L of culture medium supplemented with 10% fetal bovine serum and 0.1% DMSO.

### Application and preparation of the test substance

The definition of drug concentrations for *in vitro* evaluation was established from published pharmacokinetic studies; relating the oral dose administered to humans with the maximum plasma concentration ($C_{max}$) equivalent to this dose.
A correlation curve was outlined with this data and the linear regression for calculating the unknown plasma concentrations was performed, using the software Graphpad Instat 3.0.
After calculating the concentrations, the test substances were diluted in 100% DMSO, with stock solution concentrations being of 1 mM to ethinyl estradiol and 10 mM to Dienogest, Resveratrol and Danazol. The use solution was obtained by 1:10 serial dilutions in culture medium supplemented with 10% of fetal bovine serum and 1% of antibiotic/antimycotic. The final concentration of DMSO in the solutions was 0.1%.

### Preparation of test system Tissue collection

Endometrium tissues were obtained from 2 voluntary donors by means of videolaparoscopy, by signing the respective terms of consent and ages between 18 and 45 years. A fragment was taken from tissue samples for histological confirmation of endometriosis and the remaining was packaged in sterile flask containing culture medium DMEM/Ham's F12 (Gibco ®) with ampicillin and streptomycin (Merck®) antibiotics, and they were taken immediately to the laboratory.

### Isolation and Culture of Endometrial Cells

In the laboratory, tissue was washed with PBS pH 7.4 and split into smaller portions (1-2 mm$^3$), incubated in DMEM/Ham's F12 (Gibco ®) supplemented with Collagenase type I (Sigma ®), at a concentration of 1.2 mg/mL, during 2 hours in an oven under atmosphere of 5% $CO_2$ at 37 °C. The resulting suspension was centrifuged and re-suspended in culture medium, and it was then transferred to a 25 cm$^2$ culture bottle.

The purity of cell cultures was tested by immunocytochemistry using rabbit anti-human vimentin monoclonal primary antibody (Spring Bioscience ™) and rabbit anti-IgG secondary polyclonal antibody from goat labeled with Texas Red (Invitrogen®).

The cells were maintained and amplified in DMEM/Ham's F12 (Gibco®) 10% FBS medium, with 50 mg/mL of antibiotics ampicillin and streptomycin in culture bottle until reaching the sub-confluence (90% of confluence), and then they were transferred to 96-well plates for conducting the tests for assessing antiproliferative activity.

**Assessment of Antiproliferative Activity**

Cells were transferred to 96-well plates to a density of 10,000 cells per well in culture medium with 10% of SBF and incubated during 48 hours. After this period, the medium was removed and replaced with 200 μL of experimental medium having drug concentrations (dienogest, ethinyl estradiol and resveratrol, alone or in association) and incubation during more than 24 hours was performed. Then, MTT tetrazolium salt was added (Invitrogen) at a concentration of 1 mg/mL, according to the incubation periods described in the manufacturer's manual.

Cell proliferation was indirectly evaluated using the MTT colorimetric method (Invitrogen) and absorbance measurement at 540 nm in SpectraMax M2 plate reader-Molecular Devices with SoftMax Pro 5.2 software. By following this procedure, results of antiproliferative activity of drugs on cells can be obtained. Data were calculated with the ratio of values obtained from cultures exposed (and in different concentrations, combined or not) and cultures not exposed to drugs. The reference of 100% of cell viability, cells cultured without the presence of drugs, was considered.

**Analysis of effects of conjugated drugs**

The conjugated effects of drugs dienogest (fixed dose of 52.94 ng/mL), ethinyl estradiol and resveratrol, and the combination of dienogest (fixed dose of 52.94 ng/mL) and ethinyl estradiol were evaluated from the concentrations needed to observe equipotent effects with the drugs alone. For the analysis of this result, the equation proposed by Tallarida was used. Briefly, the concentrations of drug 1 and drug 2, when combined, that produce a given effect are respectively named Z1* and Z2*. The concentrations of drug 1 and drug 2, when isolated, that produce the same effect, are named Z1 and Z2, respectively. Hence, it is described that:

a) For additive conjugation, this ratio complies with the following equation:

$$Z1*/Z1 + Z2*/Z2 = 1$$

b) For synergistic combination, this relation complies with the following equation:

$$Z1*/Z1 + Z2*/Z2 < 1$$

c) For antagonistic conjugation, this relation complies with the following equation:

$$Z1*/Z1 + Z2*/Z2 > 1$$

These data were used to outline the $C_{max}$ curve in terms of oral dose administered. The $C_{max}$ points of the doses estimated in the study were calculated from the curve.

**EXAMPLE 6**

**EFFECTIVENESS ANALYSIS BY TALLARIDA METHOD**

From the equations described by Tallarida (Tallarida RJ. Statistical analysis of drug combinations for synergism. Pain. 1992; 49: 93-97; Tallarida RJ. Drug synergism: Its detection and applications. The Journal of Pharmacology and Experimental Therapeutics. 2001; 298: 865-872) the concentration that would produce the same effect of the conjugated drugs (equipotent effect) was calculated for each isolated drug.

The table below shows the results obtained for the conjugate (dienogest + ethinyl estradiol + resveratrol), named as sample 187C.

**Table 3A**

| Concentration values for equipotent effects between the isolated drugs and the combination between dienogest, ethinyl estradiol and resveratrol | | | | |
|---|---|---|---|---|
| Resveratrol | | Ethinyl Estradiol + Dienogest | | Effects produced by conjugated drugs |
| Oral Dose (mg) | $C_{max}$ (ng/mL) | Oral Dose (mg) | $C_{max}$ (ng/mL) | |
| 632.6 | 98.99 | 0.004 | 0.017 | 86.20% |
| 882.2 | 148.5 | 0.002 | 0.009 | 86.70% |
| 1131.8 | 197.7 | 0.01 | 0.034 | 86.30% |

In all tests, the 2 mg dose of dienogest was used, which corresponds to the concentration of 52.4 ng/mL.

The concentrations were selected from experiments that presented the best effect.

The concentrations of 197.7 ng/mL of resveratrol and 0.034 ng/mL of ethinyl estradiol were chosen as the 100% concentrations to verify the proportional concentrations, according to teachings of Tallarida. The concentrations of 98.99 ng/mL of resveratrol and 0.017 ng/mL of ethinyl estradiol were then verified, that correspond to 50% of the 100% concentrations previously elected. The concentrations of 75% of resveratrol corresponding to 148.5 ng/mL and 25% of ethinyl estradiol, corresponding to 0.009 ng/mL, were also verified.

Based on the results obtained according to the table above, the coefficient was calculated by Tallarida ratio equation in order to obtain the evaluation of the conjugated effects.

The result indicates synergism of combinations in the tests performed, especially where ethinyl estradiol/resveratrol ratio is smaller, as can be observed in the table below.

**Table 3B**

| Z1* | Z1 | Z*2 | Z2 | Conjugate Result |
|---|---|---|---|---|
| 0.017 | 0.084 | 98.99 | $1.6 \times 10^{17}$ | 0.2 |
| 0.009 | 0.076 | 148.48 | $1.5 \times 10^{16}$ | 0.12 |
| 0.034 | 0.083 | 197.97 | $1.1 \times 10^{17}$ | 0.41 |

Where: Z1 and Z2 are the concentrations of drugs isolated and Z1* and Z2 * are the concentrations of the combined compounds.

**Table 4**

| Relative mass of drugs in the combination 187C (relative to the total mass in ng/mL ($C_{Max}$). | | | |
|---|---|---|---|
| Analysis of combination 187C | | | |
| Dienogest | 52.94 | 52.94 | 52.94 |
| Resveratrol | 98.990 | 148.480 | 197.970 |
| Ethinyl Estradiol | 0.017 | 0.009 | 0.034 |
| R/E Ratio | 5.823 | 16.497 | 5.823 |
| Total mass (ng/mL) | 151.947 | 201.429 | 250.944 |
| Relative mass Dienogest | 34.84% | 26.282% | 21.10% |
| Relative Mass Resveratrol | 65.15% | 73.713% | 78.89% |

(continued)

| Relative mass of drugs in the combination 187C (relative to the total mass in ng/mL ($C_{Max}$). | | |
|---|---|---|
| Analysis of combination 187C | | |
| Relative mass Ethinyl Estradiol | 0.011% | 0.004% | 0.014% |
| Proliferation | 86.2% | 86.7% | 86.3% |
| Tallarida Coefficient | 0.2 | 0.12 | 0.41 |

**Table 5**

| Relative mass of drugs in the combination 187C (relative to the total mass in mg (oral dose) | | |
|---|---|---|
| Analysis of combination 187C | | |
| Dienogest | 2.0 | 2.0 | 2.0 |
| Resveratrol (R) | 882.2 | 1131.8 | 632.6 |
| Ethinyl Estradiol (E) | 0.002 | 0.010 | 0.004 |
| R/E Ratio | 441,100 | 113,180 | 158,150 |
| Total mass (mg) | 884.196 | 1133.847 | 634.554 |
| Relative mass Dienogest | 0.2262% | 0.1764% | 0.3152% |
| Relative Mass Resveratrol | 99.7736% | 99.8227% | 99.6842% |
| Relative mass Ethinyl Estradiol | 0.0002% | 0.0009% | 0.0006% |
| Proliferation | 86.2% | 86.7% | 86.3% |
| Tallarida Coefficient | 0.2 | 0.12 | 0.41 |

It was verified that the maintenance of ratio between the drugs directly impacts on the effect, particularly considering low doses of ethinyl estradiol.

**EXAMPLE 7**

**CELL PROLIFERATION ASSAYS**

The tables below show the obtained values of cell proliferation. These results were used to obtain the dose-response curves of each drug, alone and in combination.

**Table 6**

| Raw cell proliferation results used for the construction of the dose-response curve of resveratrol alone | | | | |
|---|---|---|---|---|
| Resveratrol alone (ng/mL) | | | | |
| | 1.980 | 19.800 | 197.970* | 1979.130 | 19797.13 |
| Proliferation | 94.8% | 90.6% | 94.7% | 93.3% | 91.1% |
| * Concentration used in the combination | | | | |

**Table 7**

| Raw cell proliferation results used for the construction of the dose-response curve of combination 187A | | | |
|---|---|---|---|
| Ethinyl estradiol + dienogest combination (187A) (ng/mL) | | | |
| Ethinyl Estradiol | 0.034 | 0.026 | 0.017 | 0.009 |

(continued)

| Raw cell proliferation results used for the construction of the dose-response curve of combination 187A | | | | |
|---|---|---|---|---|
| Ethinyl estradiol + dienogest combination (187A) (ng/mL) | | | | |
| Dienogest | 52.94 | 52.94 | 52.94 | 52.94 |
| Proliferation | 90.2% | 91.3% | 92.1% | 96.4% |

**Table 8**

| Raw cell proliferation results used for the construction of the dose-response curve of combination 187C. | | | | | | |
|---|---|---|---|---|---|---|
| Ethinyl estradiol + dienogest + resveratrol combination (187C) (ng/mL) | | | | | | |
| Ethinyl Estradiol | 0.034 | 0.026 | 0.017 | 0.009 | 0.000 | 0.034 |
| Dienogest | 52.94 | 52.94 | 52.94 | 52.94 | 52.94 | 52.94 |
| Resveratrol | 0.000 | 49.490 | 98.990 | 148.480 | 197.970 | 197.970 |
| Proliferation | 88.8% | 99.1% | 86.2% | 86.7% | 89.3% | 86.3% |

The comparative tables 6-8 show that the conjugate obtained the best result with respect to proliferation inhibition.

It shall be understood that the embodiments described above are merely illustrative and any modification to them may occur for a person skilled in the art. Therefore, the present invention should not be considered as being limited to the embodiments described in this document. The person skilled in the art can readily evaluate, by means of the teachings contained in the text and in the presented examples, the advantages of the invention, and propose modifications and equivalent alternatives to the embodiments without departing from the scope of the invention, as it is defined in the attached claims.

## Claims

1. Pharmaceutical combination to treat and/or prevent myoma and/or endometriosis, **CHARACTERIZED in that** it comprises resveratrol and progestogen.

2. Combination, according to claim 1, **CHARACTERIZED in that** it comprises 15 mg to 10,000 mg of resveratrol.

3. Combination, according to claim 1, **CHARACTERISED in that** progestogen is one or more of gestodene, drospirenone, desogestrel, dienogest or levonogestrel.

4. Combination, according to claim 3, **CHARACTERIZED in that** it comprises 35 mcg to 115 mcg of gestodene.

5. Combination, according to claim 3, **CHARACTERIZED in that** it comprises 1.5 mg to 4.5 mg of drospirenone.

6. Combination, according to claim 3, **CHARACTERIZED in that** it comprises 75 mcg to 225 mcg of desogestrel.

7. Combination, according to claim 3, **CHARACTERIZED in that** it comprises 1 mg to 4 mg of dienogest.

8. Combination, according to claim 3, **CHARACTERIZED in that** it comprises 40 to 70 mg of levonogestrel.

9. Combination, according to claim 1, **CHARACTERIZED in that** it additionally comprises an estrogen.

10. Combination, according to claim 9, **CHARACTERIZED in that** estrogen is selected from ethinyl estradiol, estradiol, estradiol valerate or mixtures thereof.

11. Combination, according to claim 10, **CHARACTERIZED in that** it comprises 0 to 40 mcg of ethinyl estradiol.

12. Combination, according to any of claims 1 to 11, **CHARACTERIZED in that** it controls the symptoms associated with myoma and/or endometriosis.

13. Combination, according to any of claims 1 to 12, **CHARACTERIZED in that** it is contraceptive or not.

14. Use of resveratrol and progestogen, **CHARACTERIZED in that** they are used in the preparation of a medicament useful in the treatment and/or prevention of myoma and/or endometriosis.

15. Use of resveratrol and progestogen, **CHARACTERIZED in that** they are used in the preparation of a medicament useful in the treatment and/or control of symptoms related to myoma and/or endometriosis.

16. Use, according to claim 15, **CHARACTERIZED in that** the symptoms include pelvic pain, dysmenorrhea, premenstrual tension, and blood escape.

17. Use, in accordance with any of claims 14 or 15, **CHARACTERIZED in that** they are useful as a contraceptive or not.

18. Pharmaceutical composition to treat and/or prevent myoma and/or endometriosis, **CHARACTERIZED in that** it comprises the association according to any of claims 1 to 12 and pharmaceutically acceptable excipients.

19. Medicament to treat and/or prevent myoma and/or endometriosis, **CHARACTERIZED in that** it comprises the association according to any of claims 1 to 12 as the active principle, and pharmaceutically acceptable excipients.

20. Kit, **CHARACTERIZED in that** it comprises 20 to 30 dosage forms containing the combination, according to any of claims 1 to 12, packed in a package with instructions for use.

21. Kit, according to claim 20, **CHARACTERIZED in that** it comprises 1 to 11 additional tablets containing resveratrol or placebos.

22. Method to treat and/or prevent myoma and/or endometriosis, **CHARACTERIZED in that** it comprises providing a combination, according to any of claims 1 to 12, to a patient in need thereof, concurrently or sequentially, during 20 to 30 days or continuously.

23. Method, according to claim 22, **CHARACTERIZED in that** it is followed by 1 to 10 days of interruption or administration of only resveratrol.

FIGURE 1

FIGURE 2A

FIGURE 2B

A                                         B

FIGURE 3

$$y = 0.6591e^{0.2625x}$$
$$R^2 = 0.9745$$

FIGURE 4

$y = 2,1789e^{0,1587x}$

$R^2 = 0,9175$

FIGURE 5

FIGURE 6

Europäisches
Patentamt

European
Patent Office

Office européen
des brevets

## EUROPEAN SEARCH REPORT

Application Number

EP 14 19 7294

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 1 462 106 A1 (PANTARHEI BIOSCIENCE BV [NL]) 29 September 2004 (2004-09-29) * claims 1-6 * | 1-23 | INV. A61K31/565 A61K31/585 A61K31/57 A61P15/00 A61K31/05 A61K31/566 A61K31/567 |
| Y | DE 44 05 899 A1 (SCHERING AG [DE]) 24 August 1995 (1995-08-24) * claims 1,5 * | 1-23 | |
| Y | US 2004/220152 A1 (BEN-MAIMON CAROLE S [US] ET AL) 4 November 2004 (2004-11-04) * claim 12 * | 1-23 | |
| Y | BRUNER-TRAN K ET AL: "Resveratrol inhibits development of experimental endometriosis and induces apoptosis of endometrial stroma", FERTILITY AND STERILITY, ELSEVIER SCIENCE INC, NEW YORK, NY, USA, vol. 92, no. 3, 1 September 2009 (2009-09-01), pages S64-S65, XP026543034, ISSN: 0015-0282, DOI: 10.1016/J.FERTNSTERT.2009.07.250 [retrieved on 2009-08-28] * abstract * | 1-23 | |
| Y | DE 10 2007 011105 A1 (BAYER SCHERING PHARMA AG [DE]) 4 September 2008 (2008-09-04) * claims 1,6,13,14 * | 1-23 | |
| Y | WO 03/041741 A1 (PANTERHEI BIOSCIENCE B V [NL]; COELINGH BENNINK HERMAN JAN TI [NL]; VI) 22 May 2003 (2003-05-22) * claims 1,3,5 * | 1-23 | |

TECHNICAL FIELDS SEARCHED (IPC)

A61K

-/--

The present search report has been drawn up for all claims

| Place of search | Date of completion of the search | Examiner |
|---|---|---|
| The Hague | 17 November 2015 | Langer, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 14 19 7294

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2006/079491 A1 (SACHSE ANDREAS [DE]) 13 April 2006 (2006-04-13) * claim 14 * | 1-23 | |
| Y | US 2005/113350 A1 (DUESTERBERG BERND [DE] ET AL) 26 May 2005 (2005-05-26) * claims 1,8,9,11,17,19 * | 1-23 | |
| Y,P | K. L. BRUNER-TRAN ET AL: "Resveratrol Inhibits Development of Experimental Endometriosis In Vivo and Reduces Endometrial Stromal Cell Invasiveness In Vitro", BIOLOGY OF REPRODUCTION, vol. 84, no. 1, 15 September 2010 (2010-09-15), pages 106-112, XP055096358, ISSN: 0006-3363, DOI: 10.1095/biolreprod.110.086744 * abstract * | 1-23 | |
| A | WO 03/017974 A1 (PANTARHEI BIOSCIENCE BV [NL]; COELINGH BENNINK HERMAN JAN TI [NL]; THI) 6 March 2003 (2003-03-06) * page 1, line 24 - line 28 * * page 14, line 20 - line 22 * | 1-23 | TECHNICAL FIELDS SEARCHED (IPC) |
| A | WO 2007/016211 A1 (WYETH CORP [US]; MCCOMAS CASEY CAMERON [US]; FENSOME ANDREW [US]; MELE) 8 February 2007 (2007-02-08) * page 1, line 26 * * page 14, line 13 - line 15 * * claims 14-18 * | 1-23 | |
| A | US 6 498 154 B1 (GRUBB GARY S [US] ET AL) 24 December 2002 (2002-12-24) * column 24, line 33 - line 39 * * column 24, line 56 - line 62 * | 1-23 | |

-/--

The present search report has been drawn up for all claims

| Place of search | Date of completion of the search | Examiner |
|---|---|---|
| The Hague | 17 November 2015 | Langer, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 14 19 7294

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | HUGO MAIA JR. ET AL: "Advantages of the association of resveratrol with oral contraceptives for management of endometriosis-related pain", INTERNATIONAL JOURNAL OF WOMEN'S HEALTH, vol. 4, 10 October 2012 (2012-10-10), pages 543-549, XP055096307, ISSN: 1179-1411, DOI: 10.2147/IJWH.S36825 * the whole document * | 1-23 | |

TECHNICAL FIELDS
SEARCHED        (IPC)

The present search report has been drawn up for all claims

| Place of search | Date of completion of the search | Examiner |
|---|---|---|
| The Hague | 17 November 2015 | Langer, Oliver |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**EP 2 965 762 A1**

## ANNEX TO THE EUROPEAN SEARCH REPORT
## ON EUROPEAN PATENT APPLICATION NO.

EP 14 19 7294

This annex lists the patent family members relating to the patent documents cited in the above-mentioned European search report.
The members are as contained in the European Patent Office EDP file on
The European Patent Office is in no way liable for these particulars which are merely given for the purpose of information.

17-11-2015

| Patent document cited in search report | | | Publication date | Patent family member(s) | | | Publication date |
|---|---|---|---|---|---|---|---|
| EP 1462106 | A1 | | 29-09-2004 | NONE | | | |
| DE 4405899 | A1 | | 24-08-1995 | AU | 1578695 | A | 04-09-1995 |
| | | | | CA | 2183544 | A1 | 24-08-1995 |
| | | | | DE | 4405899 | A1 | 24-08-1995 |
| | | | | EP | 0744943 | A1 | 04-12-1996 |
| | | | | JP | H09508911 | A | 09-09-1997 |
| | | | | NO | 963433 | A | 16-08-1996 |
| | | | | NZ | 279226 | A | 27-05-1998 |
| | | | | WO | 9522321 | A1 | 24-08-1995 |
| US 2004220152 | A1 | | 04-11-2004 | CA | 2524474 | A1 | 18-11-2004 |
| | | | | EP | 1624848 | A2 | 15-02-2006 |
| | | | | JP | 2006525358 | A | 09-11-2006 |
| | | | | US | 2004220152 | A1 | 04-11-2004 |
| | | | | WO | 2004098517 | A2 | 18-11-2004 |
| DE 102007011105 | A1 | | 04-09-2008 | AR | 065585 | A1 | 17-06-2009 |
| | | | | AU | 2008223859 | A1 | 12-09-2008 |
| | | | | BR | PI0808427 | A2 | 22-07-2014 |
| | | | | CA | 2679520 | A1 | 12-09-2008 |
| | | | | CN | 101621995 | A | 06-01-2010 |
| | | | | DE | 102007011105 | A1 | 04-09-2008 |
| | | | | EP | 2131825 | A1 | 16-12-2009 |
| | | | | JP | 2010520178 | A | 10-06-2010 |
| | | | | KR | 20090119870 | A | 20-11-2009 |
| | | | | RU | 2009136305 | A | 10-04-2011 |
| | | | | TW | 200900080 | A | 01-01-2009 |
| | | | | US | 2011003778 | A1 | 06-01-2011 |
| | | | | WO | 2008107373 | A1 | 12-09-2008 |
| WO 03041741 | A1 | | 22-05-2003 | AT | 356635 | T | 15-04-2007 |
| | | | | CA | 2467237 | A1 | 22-05-2003 |
| | | | | DE | 60218881 | T2 | 20-12-2007 |
| | | | | DK | 1443966 | T3 | 21-05-2007 |
| | | | | EP | 1443966 | A1 | 11-08-2004 |
| | | | | ES | 2281551 | T3 | 01-10-2007 |
| | | | | PT | 1443966 | E | 21-06-2007 |
| | | | | US | 2005148559 | A1 | 07-07-2005 |
| | | | | US | 2012015917 | A1 | 19-01-2012 |
| | | | | WO | 03041741 | A1 | 22-05-2003 |
| US 2006079491 | A1 | | 13-04-2006 | NONE | | | |
| US 2005113350 | A1 | | 26-05-2005 | NONE | | | |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 14 19 7294

This annex lists the patent family members relating to the patent documents cited in the above-mentioned European search report.
The members are as contained in the European Patent Office EDP file on
The European Patent Office is in no way liable for these particulars which are merely given for the purpose of information.

17-11-2015

| Patent document cited in search report | | Publication date | Patent family member(s) | | Publication date |
|---|---|---|---|---|---|
| WO 03017974 | A1 | 06-03-2003 | WO | 03017973 A1 | 06-03-2003 |
| | | | WO | 03017974 A1 | 06-03-2003 |
| WO 2007016211 | A1 | 08-02-2007 | AR | 055590 A1 | 29-08-2007 |
| | | | AU | 2006275832 A1 | 08-02-2007 |
| | | | CA | 2613484 A1 | 08-02-2007 |
| | | | CN | 101233103 A | 30-07-2008 |
| | | | EP | 1910284 A1 | 16-04-2008 |
| | | | GT | 200600335 A | 28-05-2007 |
| | | | JP | 2009502946 A | 29-01-2009 |
| | | | PE | 01822007 A1 | 06-03-2007 |
| | | | US | 2007027125 A1 | 01-02-2007 |
| | | | US | 2008064673 A1 | 13-03-2008 |
| | | | WO | 2007016211 A1 | 08-02-2007 |
| US 6498154 | B1 | 24-12-2002 | CA | 2369077 A1 | 09-11-2000 |
| | | | CN | 1349413 A | 15-05-2002 |
| | | | EP | 1173206 A1 | 23-01-2002 |
| | | | JP | 2002543156 A | 17-12-2002 |
| | | | MX | PA01011293 A | 14-07-2003 |
| | | | US | 6498154 B1 | 24-12-2002 |
| | | | WO | 0066165 A1 | 09-11-2000 |

EPO FORM P0459

For more details about this annex : see Official Journal of the European Patent Office, No. 12/82

**EP 2 965 762 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HARIKUMAR et al.** Resveratrol - A multitargeted agent for age-associated chronic diseases. *Cell Cycle,* 15 April 2008, vol. 7 (8), 1020-1035
- **CHEN.** Effects of genistein, resveratrol, and quercetin on steroidogenesis. *Journal of Endocrinology,* 2007, vol. 192, 527-537
- **TALLARIDA RJ.** Statistical analysis of drug combinations for synergism. *Pain,* 1992, vol. 49, 93-97
- **TALLARIDA RJ.** Drug synergism: Its detection and applications. *The Journal of Pharmacology and Experimental Therapeutics,* 2001, vol. 298, 865-872